# EUROPÄISCHE PATENTANMELDUNG

(11) **EP 1 391 182 A2**
(43) Veröffentlichungstag der Anmeldung: **25.02.2004**
(21) Anmeldenummer: 03018243.0
(22) Anmeldetag: 11.08.2003
(51) Int. Cl.: A61C 1/08

(54) **Funktionshandstück mit einem Lichtabstrahlelement an seinem vorderen Ende**

(30) Priorität: 22.08.2002 DE 10238555
(71) Anmelder: Kaltenbach & Voigt GmbH & Co. KG, 88400 Biberach (DE)
(72) Erfinder: Schmid, Gerhard, 88441 Mittelbiberach (DE); Löhn, Gerd, 88400 Biberach-Rissegg (DE); Liebhardt, Franz, 88416 Ochsenhausen (DE); Mösslang, Hubert, 89610 Oberdischingen (DE)
(74) Vertreter: Schmidt-Evers, Jürgen, Dipl.-Ing.

(57) **Zusammenfassung**

Funktionshandstück (1) mit einem länglichen Handstückkörper (2), der an seinem hinteren Ende ein Verbindungselement zum Verbinden mit einer flexiblen Versorgungsleitung aufweist, und der an seinem vorderen Ende ein Lichtabstrahlelement (3) aus lichtdurchlässigem Material zum Beleuchten der Behandlungsstelle und im Lichtabstrahlelement (3) eine Auslassöffnung (25) für ein Medium wie Wasser oder Luft oder Spray aufweist, wobei das Lichtabstrahlelement (3) einen vorderen Bereich des Funktionshandstücks (1) bildet und lösbar mit dem übrigen Bereich des Funktionshandstücks (1) verbunden ist, und wobei das Lichtabstrahlelement (3) eine Steckfassung (3a) für das Lichtabstrahlelement (3) aufweist. Um das Funktionshandstück bezüglich der Ausleuchtung der Behandlungsstelle zu verbessern, ist das Lichtabstrahlelement (3) durch eine Verrastungsvorrichtung (6) mit dem übrigen Bereich des Funktionshandstücks (1) verbunden, wobei die Verrastungsvorrichtung (6) eine mittelbar oder unmittelbar am Lichtabstrahlelement (3) angeordnete Verrastungsnase (6d) aufweist, die radial nach innen einfederbar ist und selbsttätig hinter einer Verrastungskante (6e) an der Steckausnehmung (6c) ausfederbar ist.

## Beschreibung

Die Erfindung betrifft ein Funktionshandstück nach dem Oberbegriff des Anspruchs 1 oder 3.

Bei ärztlichen oder zahnärztlichen Handstücken unterscheidet man zwischen solchen, die mit einem Werkzeug zur Behandlung des menschlichen oder tierischen Körpers bestückt oder bestückbar sind und mit denen mechanisch auf den menschlichen oder tierischen Körpers eingewirkt wird, und solchen Handstücken, die dazu dienen, die Behandlungsstelle des menschlichen oder tierischen Körpers in einem operationsfähigen Zustand zu halten, zum Beispiel Wasser und/oder Luft zur Kühlung und Reinigung der Behandlungsstelle zuzuführen, oder Licht zur Behandlungsstelle zuzuführen, oder Körperflüssigkeit, z.B. Speichel oder Blut, von der Behandlungsstelle abzuführen oder die Behandlungsstelle zu sondieren. Im vorliegenden medizinischen Fachbereich wird die erste Art Handstücke mit Arbeitshandstücke und die zweite Art mit Funktionshandstücke bezeichnet. Bei einem Funktionshandstück kann es sich somit um ein Spül- und/oder Blashandstück, Beleuchtungshandstück oder ein Sondenhandstück handeln.

Ein Funktionshandstück in Form eines Spül- und Blashandstücks ist z.B. in der DE 35 00 085 C2 beschrieben. Dieses zahnärztliche Funktionshandstück dient dazu, Wasser und/oder Luft oder Licht der Behandlungsstelle zuzuführen. Dieses Funktionshandstück weist ein längliches Griffteil auf, mit dessen vorderem Ende eine Kanüle lösbar verbunden ist, die an ihrem vorderen Ende ein Lichtabstrahlelement aufweist, dem Licht durch einen Lichtleiter oder direkt durch eine Lampe zuführbar ist, und das das Licht im Funktionsbetrieb nach vorne auf die Behandlungsstelle abstrahlt. Das Lichtabstrahlelement ist von einem Schutzrohr der Kanüle umgeben, und es weist an seinem freien Ende eine zentrale Düse für Wasser und eine die Düse umgebende zweite Ringdüse für Luft auf, die in ein gemeinsames Düsenloch münden. Das Düsenloch ist von einer Buchse umgeben, die in einem vorderen Aufnahmeloch des Lichtabgabekörpers fest eingesetzt ist. Das Lichtabstrahlelement weist somit an seinem vorderen freien Ende eine Ringstirnfläche auf, aus der das Licht ringförmig nach vorne austritt.

DE 100 45 115 A1 zeigt ein medizinisches oder dentalmedizinisches Handstück, dessen vorderer Endbereich durch ein Lichtabstrahlelement gebildet ist, das durch eine Steckfassung mit Klemmverbindung mit dem Handstück lösbar verbunden ist.

Aus WO 99/47068 A ist ein Saughandstück in einer Vielzahl von Ausführungsbeispielen beschrieben. Fig. 15-19 zeigen ein Lichtabstrahlelement, das durch eine Steckfassung mit einer Verrastungsvorrichtung lösbar mit dem Saughandstück verbunden ist.

Die Behandlungsmaßnahmen des menschlichen oder tierischen Körpers können sowohl mit dem Funktionshandstück selbst als auch in Verbindung mit einem weiteren Arbeitshandstück vielseitig sein, wobei eine gute Beleuchtung der Behandlungsstelle erwünscht ist, so dass der Behandler sowohl die Behandlungsstelle selbst als auch das Behandlungsergebnis deutlich beobachten kann.

Der Erfindung liegt deshalb die Aufgabe zu Grunde, ein Funktionshandstück der eingangs angegebenen Arten bezüglich der Ausleuchtung der Behandlungsstelle zu verbessern. Insbesondere soll eine vermehrt räumliche Beleuchtung der Behandlungsstelle möglich sein.

Diese Aufgabe wird durch die Merkmale des Anspruchs 1 oder 3 gelöst. Vorteilhafte Weiterbildungen der Erfindung sind in den Unteransprüchen beschrieben.

Beim erfindungsgemäßen Mehrfunktionshandstück nach Anspruch 1, der gegen DE 100 45 115 A abgegrenzt ist, und beim erfindungsgemäßen Funktionshandstück nach Anspruch 3, der gegen WO 99/47068 A abgegrenzt ist, bildet das Lichtabstrahlelement den vorderen Endbereich des Handstücks, und es ist lösbar verbunden. Hierbei wird die angestrebte bessere räumliche Beleuchtung der Behandlungsstelle dadurch erreicht, dass das Licht im Funktionsbetrieb nicht nur nach vorne sondern auch seitlich abstrahlt und deshalb die Behandlungsstelle räumlich beleuchtet wird, wobei auf Grund der beträchtlichen Größe des Lichtabstrahlelements eine Schattenbildung wesentlich reduziert ist. Die lösbare Verbindung des Lichtabstrahlelements ermöglicht es, diesen z.B. nach einer Beeinträchtigung in seiner Lichtabstrahlung auszutauschen, so dass das Funktionshandstück in zeitsparender und materialsparender Weise in einem guten Funktionszustand gehalten werden kann. Bei einer Beeinträchtigung der Lichtabstrahlung, die z.B. dann vorliegt, wenn die Lichtabstrahlfläche verkratzt ist, kann das Lichtabstrahlelement durch ein neues Lichtabstrahlelement ausgetauscht werden, so dass sich der Austausch mit geringem Zeit- und Materialaufwand beheben lässt. Die lösbare Verbindung ist vorzugsweise eine Schnellverbindung, insbesondere mit einer Verrastungsvorrichtung, die nicht nur ein schnelles Verbinden, sondern auch eine sichere und unverlierbare Verbindung ermöglicht.

Das Licht kann dem Lichtabstrahlkörper durch einen oder zwei sich von hinten nach vorne erstreckende Lichtleiter zugeführt werden, der bzw. die vorzugsweise exzentrisch angeordnet sind, so dass eine Wasserleitung so angeordnet werden kann, dass sie am Lichtabstrahlelement koaxial angeschlossen ist oder diesen koaxial durchsetzt.

Die erfindungsgemäße Ausgestaltung eignet sich somit in vorteilhafter Weise für Spül- bzw. Spritz- und Blashandstücke, oder dergleichen oder Saughandstücke oder Sondenhandstücke mit einer Bildaufnahmeeinrichtung zum Aufnehmen von Bildern der Behandlungsstelle.

In den Unteransprüchen sind Merkmale enthalten, die die Zugänglichkeit des Funktionshandstücks zur Behandlungsstelle verbessern, zu kleinen und kostengünstig herstellbaren Ausgestaltungen führen und eine sichere Funktion und gute Handhabung gewährleisten.

In der DE 33 37 166 A1 ist ein zahnärztliches Spritzhandstück mit einer Kanüle beschrieben, die durch eine sogenannte Steck-/Drehkupplung mit dem Handstückkörper lösbar verbunden ist. Die Kanüle ist zumindest im hinteren Bereich massiv ausgebildet, wobei eine das Kupplungselement aufweisende Kanülenbasis einteilig mit dem Kanülenmantel ausgebildet ist. Hierdurch ist die Kanüle zwar stabil, jedoch ist sie materialintensiv und von verhältnismäßig schwerer Bauweise, was vermieden werden soll.

Der Erfindung liegt deshalb im weiteren die Aufgabe zugrunde, eine Kanüle der im Oberbegriff des Anspruches 13 angegebenen Art so auszugestalten, dass sie bei Gewährleistung einer materialsparenden und leichten Bausweise stabil ist.

Diese Aufgabe wird durch die Merkmale des Anspruchs 13 gelöst. Weiterbildungen der Erfindung sind in zugehörigen Unteransprüchen beschrieben.

Bei dieser Kanüle sind die Kanülenbasis und der Kanülenmantel zweiteilig ausgebildet. Außerdem ist die Kanülenbasis mit einem Stützarm nach vorne verlängert, der in einem nach vorne gerichteten Abstand von der Kanülenbasis eine quer gerichtete Abstützung zwischen diesen Teilen bildet.

Aufgrund der Zweiteiligkeit lässt sich die Kanüle in einfacher Weise hohl und dünnwandig herstellen, wodurch der Materialverbrauch und das Gewicht verringert werden können. Trotzdem ist die erfindungsgemäße Kanüle stabil, weil der Kanülenmantel durch den Stützarm seitlich gestützt ist, der durch eine nach vorne gerichtete Verlängerung der Kanülenbasis gebildet ist.

Diese erfindungsgemäße Ausgestaltung eignet sich sowohl für gerade als auch seitlich abgewinkelte oder seitlich gekrümmte Kanülen. In dem Fall, in dem sich wenigstens eine Medienleitung durch die Kanüle erstreckt, ist es vorteilhaft, den Stützarm mit einer einseitigen Wandung oder schalenförmig auszubilden, wodurch ein Hohlraum geschaffen wird, in dem die wenigstens eine Medienleitung Platz hat. Bei einer gekrümmten oder abgewinkelten Kanüle ist die seitliche Wandung vorzugsweise an der Innenseite der Krümmung bzw. Abwinklung angeordnet.

Zur Verbindung der Kanülenbasis mit dem Stützarm kann eine Steckverbindung mit einer Steckausnehmung vorgesehen sein, die einen Steckzapfen des jeweiligen Steckverbindungsteils aufnimmt.

Nachfolgend werden vorteilhafte Ausgestaltungen der Erfindung anhand eines bevorzugten Ausführungsbeispiels näher erläutert. Es zeigt:

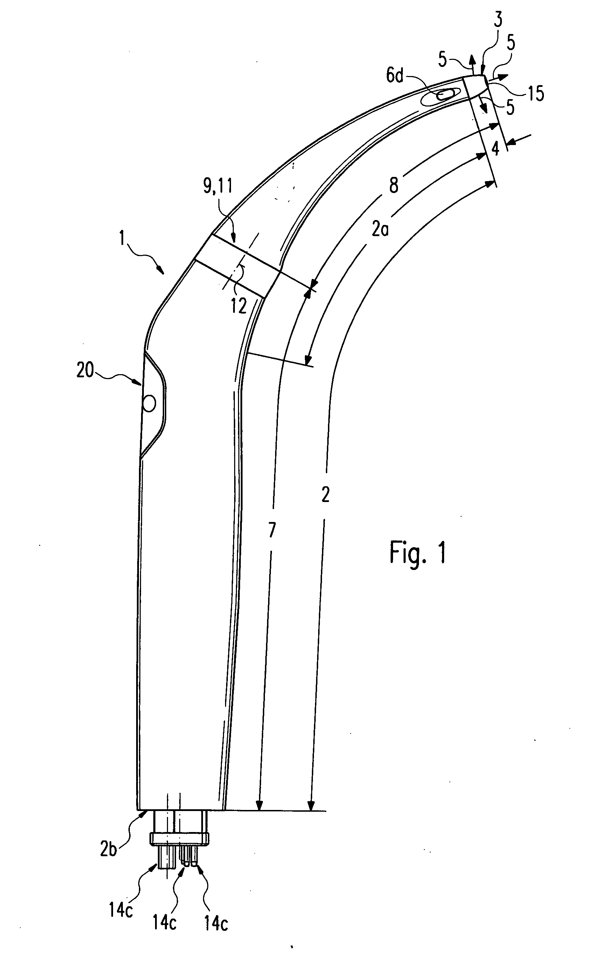

- Fig. 1: ein erfindungsgemäßes Mehrfunktionshandstück in der Seitenansicht;

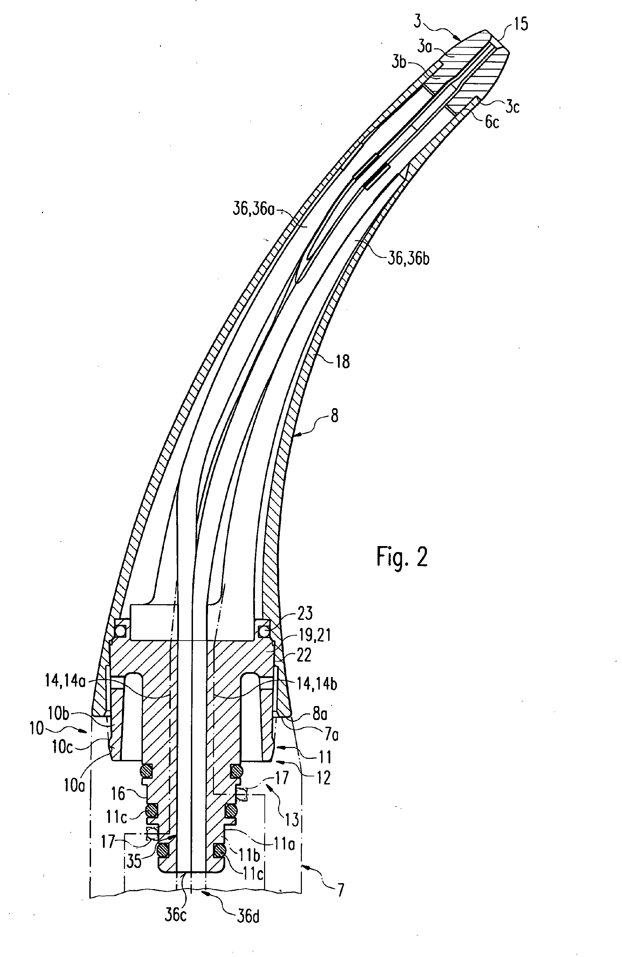

- Fig. 2: eine Kanüle des Mehrfunktionshandstücks im axialen Schnitt und in vergrößerter Darstellung;

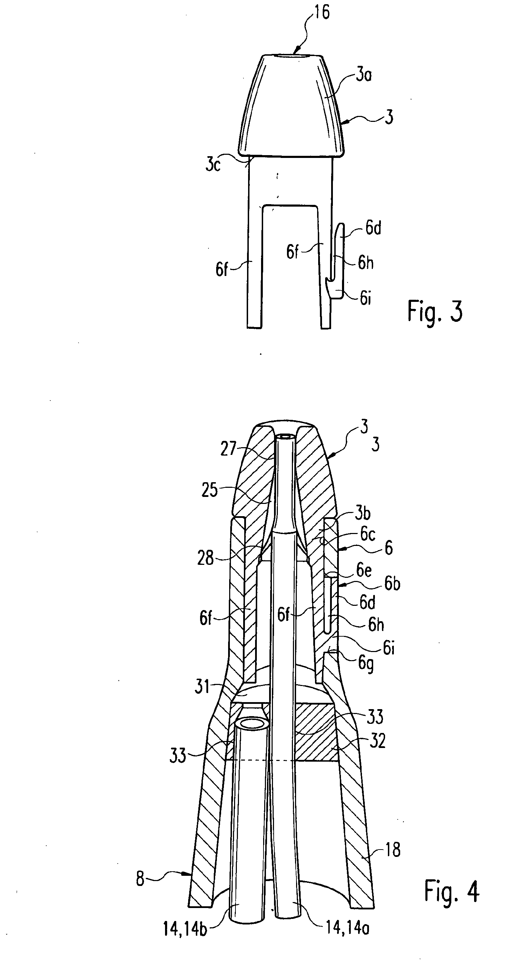

- Fig. 3: den vorderen Endbereich der Kanüle in weiter vergrößerter Darstellung;
- Fig. 4: ein Lichtabgabeteil des Mehrfunktionshandstücks in der Seitenansicht als Einzelteil.

Beim vorliegenden Ausführungsbeispiel dient das in seiner Gesamtheit mit 1 bezeichnete Funktionshandstück dazu, ein oder mehrere Behandlungsmedien, hier Wasser und/oder Luft bzw. Spray und/oder Licht einer mit dem Funktionshandstück 1 zu behandelnden Behandlungsstelle zuzuführen. Das Funktionshandstück 1 ist von länglicher Bauform, und es weist einen Handstückkörper 2 auf, der sich gerade erstrecken kann oder einen vorderen Handstückabschnitt 2a aufweisen kann, der mit seinem vorderen Ende seitlich abgewinkelt oder gekrümmt ist, wie es das Ausführungsbeispiel zeigt. Vom vorderen Ende des Handstückkörpers 2 erstreckt sich ein Lichtabstrahlelement 3 noch vorne, das einen Kopfabschnitt 4 des Funktionshandstücks 1 bildet und im Funktionsbetrieb durch Lichtstrahlen 5 verdeutlichtes Licht nach vorne und allseitig seitlich abstrahlt und auf Grund der Abstrahlfläche beträchtlicher Größe die Behandlungsstelle weitgehend schattenfrei ausleuchtet, was zumindest für die nähere Umgebung des Lichtabstrahlelements 3 gilt. Beim Ausführungsbeispiel besteht das Lichtabstrahlelement 3 aus Kunststoff oder Glas bzw. Quarz, und es weist einteilig einen den Kopfabschnitt 4 bildenden Lichtabstrahlkörper 3a und einen Verbindungskörper 3b auf, der beim Ausführungsbeispiel durch einen sich vom Lichtabstrahlkörper 3a zentrisch nach hinten erstreckenden Steckzapfen gebildet ist. Der Kopfabschnitt 4 bzw. Lichtabstrahlkörper 3a liegt mit einer den Verbindungskörper 3b verjüngenden Schulterfläche 3c am vorderen Ende des Handstückkörpers 2 an.

Der Verbindungskörper 3b ist Teil einer Verbindungsvorrichtung 6, die vorzugsweise durch eine Schnellverbindungsvorrichtung, insbesondere eine Steckfassung 6a mit einer Verrastungsvorrichtung 6b, gebildet ist, die weiter unten noch näher beschrieben wird. Der zapfenförmige Verbindungskörper 3b ist in eine vorderseitig ausmündende passende Steckausnehmung 6c im Handstückkörper 2 eingesteckt und gegen ein unbeabsichtigtes Lösen gesichert, insbesondere verrastet.

Im Rahmen der Erfindung kann der Handstückkörper 2 einteilig ausgebildet sein. Beim Ausführungsbeispiel ist er mit einem hinteren Griffteil 7 und einer sich von dessen vorderen Ende nach vorne erstreckenden Kanüle 8 quergeteilt und zweiteilig ausgebildet, wobei zur Verbindung der Kanüle 8 mit dem Griffteil 7 ebenfalls eine Verbindungsvorrichtung 9, insbesondere eine Schnellverbindungsvorrichtung, vorgesehen ist. Diese kann durch eine Steckfassung 11 mit einer Steckausnehmung 11a und einem darin passend einsteckbaren Steckzapfen 11b gebildet sein. Beim Ausführungsbeispiel mündet die Steckausnehmung 11a im Bereich einer ebenen und quer verlaufenden Stirnfläche 7a aus dem Griffteil 7 aus, und der Steckzapfen 11b erstreckt sich von der Kanüle 8 im Bereich einer zur Stirnfläche 7a parallelen Stirnfläche 8a der Kanüle 8 nach hinten. Die Steckausnehmung 11a und der Steckzapfen 11b sind vorzugsweise kreisrund oder zylindrisch oder stufenzylindrisch ausgebildet. Hierdurch bilden sie ein Drehlager 12 für die Kanüle 8, in dem diese frei drehbar gelagert ist. Zur axialen Sicherung der Kanüle 8 in der Steckfassung 11 ist eine beim manuellen Zusammenstecken selbsttätig einfedernde und beim Auseinanderziehen selbsttätig ausfedernde Verrastungsvorrichtung 10 vorgesehen, mit z.B. einer oder zwei einander gegenüberliegenden Rastnasen 10a an vom Verbindungskörper 3b nach hinten ragenden Federarmen 10b, wobei die Rastnasen 10a eine vorzugsweise ringförmige Rastkante 10c in der Steckausnehmung 11a hintergreifen und beim manuellen Auseinanderziehen ausfedern. Hierdurch ist eine Dreh/Steckkupplung mit einer Verrastungsvorrichtung gebildet, die manuell überdrückbar ist.

Das Funktionshandstück 1 weist wenigstens eine Mediumleitung 14 für ein strömungsfähiges Medium, zum Beispiel Wasser oder Luft oder Spray, auf, die sich von hinten längs durch das Funktionshandstück 1 zu einer im Lichtabgabeelement 3, vorzugsweise stirnseitig und mittig, ausmündenden Mediendüse 15 erstreckt. Dabei durchsetzt die Mediumleitung 14 eine vorzugsweise hohlzylindrische Teilungsfuge 16 zwischen den Kupplungsteilen 11a, 11b Z-förmig. Der radiale Mediumleitungsabschnitt durchsetzt die Teilungsfuge 16 im Bereich einer Ringnut 17, die sich in der Innenmantelfläche der Steckausnehmung 11a oder in der Außenmantelfläche des Steckzapfens 11b befinden kann. Hierdurch ist bei einer kreisrunden Querschnittsausbildung der Steck/Drehkupplung 13 eine Drehbarkeit über 360° hinaus bei permanenter Durchströmung des Mediums durch die Kupplung gewährleistet. Der Durchgang der Medienleitung 14 ist durch die Teilungsfuge 16 axial durch auf beiden Seiten in Ringnuten der Ausnehmung 11a oder des Zapfens 11b angeordnete Dichtringe 11c abgedichtet.

Beim Ausführungsbeispiel sind zwei Medienleitungen 14a, 14b in vorbeschriebener Weise ausgebildet, nämlich eine für Wasser und eine für Luft beziehungsweise für Druckluft, die im Bereich der Mediendüse 15 zusammengeführt sind und im Funktionsbetrieb einen Spray bilden, der aus der Mediendüse 15 vorzugsweise nach vorne austritt.

Das gewünschte Medium ist durch eine manuelle Betätigung von Drucktasten 20 einund ausschaltbar, die im vorderen Bereich des Griffabschnitts 7 angeordnet sind.

Am hinteren Ende weist der Handstückkörper 2 ein Kupplungselement 2b auf, mit dem er mit einer flexiblen Versorgungsleitung verbindbar, insbesondere verschraubbar ist. Zur lösbaren Verbindung der wenigstens einen Mediumleitung 14 mit zugehörigen Medienleitungsabschnitten in der Versorgungsleitung ist ein Leitungskupplungselement 14c vorgesehen.

Die wenigstens eine Mediumleitung 14 kann sich in der Kanüle 8 als Kanal oder wenigstes abschnittweise als Schlauch oder Rohr erstrecken. Beim Ausführungsbeispiel besteht die Kanüle 8 aus einem hülsenförmigen Kanülenmantel 18, in den von hinten eine Kanülenbasis 19 in Form eines zylindrischen Steckzapfens 21 in eine rückseitige Steckausnehmung 22 des Kanülenmantels 18 fest eingesetzt und abgedichtet ist, zum Beispiel mittels eines O-Ringes 23, der in einer Ringnut, insbesondere des Steckzapfens 21, sitzt und die Teilungsfuge abdichtet. Die Kanülenbasis 19 kann in den Kanülenmantel 18 z.B. durch Preßsitz, Kleben oder Verrasten befestigt sein.

Die Medienleitungen 14a, 14b erstrecken sich nach ihrem Z-förmigen Durchgang durch die Teilungsfuge axial nach vorne, und sie sind mit Steckverbindungselementen an der Vorderseite der Kanülenbasis 19 verbunden, die mit den Medienschläuchen oder -rohren zusammengesteckt sind. Diese erstrecken sich nach vorne, wobei die Wasserleitung sich in ein zentrales Durchgangsloch 25 im Lichtabstrahlelement 3 erstreckt, das in seinem hinteren Bereich in seinem Querschnitt beträchtlich größer bemessen ist, als die Wasserleitung 14a. Das Durchgangsloch 25 bzw. der hierdurch gebildete Ringkanal zwischen dem Lichtabstrahlelement 3 und der Wasserleitung 14a verjüngt sich nach vorn, so dass im vorderen Endbereich das Durchgangsloch 25 die Wasserleitung 14a mit einem geringen Ringspalt 27 umgibt. Zur Zentrierung der in einem geringen Abstand von der Stirnseite des Lichtabgabeelements 3 endenden Wasserleitung 14a ist im mittleren Bereich des Lichtabgabeelements 3 ein ringförmiges Zentrierelement 28 vorgesehen, das mit seinem Außenumfang an der Innenmantelfläche des Durchgangslochs 25 abgestützt ist und mit seinem Innenumfang die Wasserleitung 14a eng umschließt und somit zentriert.

Die Luftleitung 14b endet in einem nach hinten gerichteten Abstand vom vorderen Ende der Wasserleitung 14a in den vergrößerten Ringquerschnitt 25. Beim Ausführungsbeispiel mündet die Luftleitung 14b in den zwischen dem Kanülenmantel 18 und der Wasserleitung 14a vorhandenen Ringraum 31, der im Bereich des vorderen Endes der Luftleitung 14b durch eine Scheibe 32 axial unterteilt und abgedichtet ist, so dass die Druckluft nicht in den hinteren Bereich des Ringraums 31 gelangen kann. Die Scheibe 32 ist dicht eingesetzt, und sie wird von der Wasserleitung 14a und der Luftleitung 14b in einem oder zwei benachbarten Löchern 33 abgedichtet durchsetzt. Die Luftleitung 14b endet vor dem Lichtabstrahlelement 3. Das Durchgangsloch 25 schließt sich an dem Ringraum 31 an. Das Zentrierelement 28 ist durch nicht dargestellte achsparallele Löcher durchlässig für die Luft.

Hinter dem Lichtabstrahlelement 3 ist eine Lichtzuführungsvorrichtung 35 angeordnet, die Licht in das Lichtabstrahlelement 3 einstrahlt. Beim Ausführungsbeispiel ist wenigstens ein Lichtleiter 36 vorgesehen, der sich von hinten zum Lichtabstrahlelement 3 erstreckt und in dem Licht von hinten eingestrahlt wird. Der Lichtleiter 36 kann am hinteren Ende der Kanüle 8 beginnen, hier am hinteren Ende des Steckzapfens 11b. Seine hintere vorzugsweise ebene Stirnfläche 36c ist eine Lichteinspeisefläche. Die Lichteinspeisung in den Lichtleiter 36 kann durch eine im Griffabschnitt 7 angeordnete Lampe erfolgen. Es kann aber auch ein hinterer, lichtzuführender Lichtleiter 36d im Griffteil 7 angeordnet sein und sich bis zum Lichtleiter 36 erstrecken. Es ist vorteilhaft, den Lichtleiter 36 stumpf am Lichtabstrahlelement 3 enden zu lassen, hier an der Rückfläche des zapfenförmigen Verbindungskörpers 3b. Beim Ausführungsbeispiel sind zwei entsprechende Lichtleiter 36a, 36b nebeneinander liegend angeordnet. Die Kanüle 8 und der Kopfabschnitt 4 konvergieren in ihren Querschnittsgrößen in Richtung nach vorne, vorzugsweise kontinuierlich.

Die Verrastungsvorrichtung 6b ist manuell lösbar, und es ist deshalb möglich, das Lichtabstrahlelement 3 zu demontieren und gegen ein anderes bzw. neues Lichtabstrahlelement 3 auszutauschen, z.B. dann, wenn die Abstrahlfläche beeinträchtigt ist, z.B. verkratzt ist, was bei der Zusammenarbeit mit dem Bohrer eines Arbeitshandstücks leicht passieren kann.

Die Verrastungsvorrichtung 6b weist eine Verrastungsnase 6d auf, die beim Zusammenstecken der Verrastungsteile durch eine Kraft einer Feder oder aufgrund ihrer Materialelastizität selbsttätig in ihre Verrastungsstellung ausfedert, in der sie eine Verrastungskante 6e hintergreift. Beim Ausführungsbeispiel ist die Verrastungsnase 6d an einem am Verbindungskörper 3b exzentrisch angeordnet und nach hinten abstehenden Federarm 6f angeformt, der beim Einstecken des Verbindungskörpers 3b einfedert und mit der Verrastungsnase 6d selbsttätig ausfedert, wenn diese in den Bereich eines nach außen durchgehenden Verrastungsloches 6g im Hülsenmantel 18 gelangt, dessen vordere Innenkante die Verrastungskante 6e bildet. Die Form und Größe des Verrastungsloches 6g ist mit einem geringen Bewegungsspiel an die Form und Größe der Verrastungsnase 6d angepasst, so dass diese das Verrastungsloch 6g im Wesentlichen ausfüllt. In der Verrastungsstellung ist die Verrastungsnase 6d von außen zugänglich und sie kann durch das Verrastungsloch 6g in ihre Freigabestellung bewegt werden, z.B. mit einem Finger oder Fingernagel. Dem Federarm 6f gegenüberliegend kann ein zweiter Arm 6f vorgesehen sein, der die Steckführung verbessert.

Zur Vereinfachung der Lösung der Verrastung ist es vorteilhaft, die Verrastungsnase 6d mit einem Hinterschnitt 6h in ihrem Fußbereich auszubilden, wobei der Hinterschnitt 6h sich bis zu einem Verbindungssteg 6i im hinteren Bereich der Verrastungsnase 6d erstreckt. Hierdurch wird ein Einfedern des vorderen Bereichs der Verrastungsnase 6d durch Einbiegen ermöglicht, wodurch das Bewegen der Verrastungsnase 6d über die Verrastungskante 6e hinaus vereinfacht wird.

Bei einer zweiteiligen Ausgestaltung der Kanüle 8 mit dem Kanülenmantel 18 und der Kanülenbasis 19 ist es vorteilhaft, die Kanülenbasis 19 mit einem Stützarm 19a nach vorne zu verlängern, der in einem nach vorne gerichteten Abstand a von der Kanülenbasis 19 eine seitliche bzw. quer gerichtete Abstützung zwischen diesen Teilen schafft. Dies ist insbesondere bei einer verhältnismäßig kurzen Steckfassung für die Kanülenbasis 19 mit Rastfederarmen 10a vorteilhaft.

Beim Ausführungsbeispiel ist der Stützarm 19a zweiteilig mit der Kanülenbasis 19 ausgebildet und an deren vorderen Ende befestigt, z. B. dadurch, daß ein Fußabschnitt 19b oder Flansch an der Stirnseite befestigt ist, z. B. durch nicht dargestellte Schrauben, Kleben oder Schweißen. Dabei kann der Fußabschnitt 19b in einer Ausnehmung 19c eingesetzt sein, z. B. eingepreßt sein.

Eine zusätzliche Abstützung wird erzielt, wenn das vordere Ende des Stützarms 19a an einer Stufenfläche 19d des Kanülenmantels 18 anliegt. Der Stützarm 19a kann mit seinem Fußabschnitt 19b winkelförmig geformt sein, um Raum für den oder die Lichtleiter 36 zu schaffen.

Ein Schalter zum Einschalten des Lichts ist z.B. am Funktionshandstück 1 oder an einem Fußschalter (nicht dargestellt) vorgesehen.

Die Einzelteile des Funktionshandstücks 1, insbesondere die von außen zugänglichen Teile, bestehen aus einem Material, das auch bezüglich Desinfektionsmitteln oder auch Sterilisationsmitteln korrosionsbeständig ist, vorzugsweise aus Kunststoff und/oder Metall.

## Patentansprüche

1. Funktionshandstück (1) mit
- einem länglichen Handstückkörper (2),
- der an seinem hinteren Ende ein Verbindungselement zum Verbinden mit einer flexiblen Versorgungsleitung aufweist,
- und der an seinem vorderen Ende ein Lichtabstrahlelement (3) aus lichtdurchlässigem Material zum Beleuchten der Behandlungsstelle
- und im Lichtabstrahlelement (3) eine Auslassöffnung (15) für ein Medium wie Wasser oder Luft oder Spray aufweist,
wobei das Lichtabstrahlelement (3) einen vorderen Bereich des Funktionshandstücks (1) bildet und lösbar mit dem übrigen Bereich des Funktionshandstücks (1) verbunden ist,
und wobei das Lichtabstrahlelement (3) eine Steckfassung (6a) für das Lichtabstrahlelement (3) aufweist,
**dadurch gekennzeichnet,**
**daß** das Lichtabstrahlelement (3) durch eine Verrastungsvorrichtung (6b) mit dem übrigen Bereich des Funktionshandstücks (1) verbunden ist,
wobei die Verrastungsvorrichtung (6b) eine mittelbar oder unmittelbar am Lichtabstrahlelement (3) angeordnete Verrastungsnase (6d) aufweist, die radial nach innen einfederbar ist und selbsttätig hinter einer Verrastungskante (6e) an der Steckfassung (6a) ausfederbar ist.

2. Funktionshandstück nach Anspruch 1,
**dadurch gekennzeichnet,**
**dass** die Verrastungsnase (6d) zum Lösen durch ein von außen zugängliches Loch (6g) im übrigen Bereich einfederbar ist.

3. Funktionshandstück (1) mit
- einem länglichen Handstückkörper (2),
- der an seinem hinteren Ende ein Verbindungselement zum Verbinden mit einer flexiblen Versorgungsleitung aufweist,
- und der an seinem vorderen Ende ein Lichtabstrahlelement (3) aus lichtdurchlässigem Material zum Beleuchten der Behandlungsstelle
- und im Lichtabstrahlelement (3) eine Öffnung (15) für ein Medium wie Wasser oder Luft oder Spray aufweist,
wobei das Lichtabstrahlelement (3) einen vorderen Bereich des Funktionshandstücks (1) bildet und durch eine Steckfassung (6a) mit einer Verrastungsvorrichtung (6b) lösbar mit dem übrigen Bereich des Funktionshandstücks (1) verbunden ist,
und wobei die Verrastungsvorrichtung (6b) eine mittelbar oder unmittelbar am Lichtabstrahlelement (3) angeordnete Verrastungsnase (6d) aufweist,
**dadurch gekennzeichnet,**
**dass** die Verrastungsnase (6d) selbsttätig hinter einer Verrastungskante (6e) an der Steckfassung (6a) in seine Verrastungsstellung ausfederbar ist und zum Lösen durch ein Loch (6d) im übrigen Bereich von außen zugänglich ist.

4. Funktionshandstück nach einem der vorherigen Ansprüche,
**dadurch gekennzeichnet,**
**dass** die Außenmantelfläche des Lichtabstrahlelements (3) und des daran anschließenden übrigen Bereichs des Funktionshandstücks (1) stufenlos ineinander übergehen.

5. Funktionshandstück nach einem der vorherigen Ansprüche,
**dadurch gekennzeichnet,**
**dass** vom Lichtabstrahlelement (3) ein Steckzapfen (3b) nach hinten absteht, der in einer Steckausnehmung (3a) im sich anschließenden übrigen Bereich des Funktionshandstücks (1) sitzt.

6. Funktionshandstück nach Anspruch 5,
**dadurch gekennzeichnet,**
**dass** das Lichtabstrahlelement (3) mit einer den Steckzapfen (3b) verjüngenden Stufenfläche (3c) am übrigen Bereich anliegt.

7. Funktionshandstück nach einem der vorherigen Ansprüche,
**dadurch gekennzeichnet,**
**dass** die Verrastungsnase (6d) an einem nach hinten abstehenden Federarm (3b) angeordnet ist.

8. Funktionshandstück nach einem der vorherigen Ansprüche,
**dadurch gekennzeichnet,**
**dass** der übrige Bereich durch eine Kanüle (8) gebildet ist, die vorzugsweise durch eine Schnellverbindung lösbar mit einem Griffteil (7) verbunden ist.

9. Funktionshandstück nach Anspruch 8,
**dadurch gekennzeichnet,**
**dass** die Kanüle seitlich gekrümmt oder abgewinkelt ist.

10. Funktionshandstück nach Anspruch 8 oder 9,
**dadurch gekennzeichnet,**
**dass** die Kanüle (8) um die Längsachse des Funktionshandstücks (1) drehbar gelagert ist.

11. Funktionshandstück nach einem der Ansprüche 8 bis 10,
**dadurch gekennzeichnet,**
**dass** die Kanüle (8) durch eine Steck/Drehkupplung verbunden ist.

12. Funktionshandstück nach Anspruch 11,
**dadurch gekennzeichnet,**
**dass** eine oder mehre Medienleitungen (14, 14a, 14b) eine hohlzylindrische Teilungsfuge (16) der Steck/Drehkupplung Z-förmig durchsetzen und/oder wenigstens ein Lichtleiter (36) die Steck/Drehkupplung axial durchsetzt und sich zum Lichtabstrahlelement (3) erstreckt.

13. Kanüle (8) für ein Funktionshandstück (1) mit einem Kanülenmantel (18), der an seinem hinteren Ende eine Kanülenbasis (19) und an seinem vorderen Ende eine Öffnung (15) aufweist, wobei der Kanülenbasis (19) ein Verbindungsvorrichtungsteil zum Verbinden der Kanüle (8) mit einem Griffteil (7) des Handstücks (1) angeordnet ist und wenigstens eine Medienleitung sich durch die Kanülenbasis (19) bis zur Öffnung (15) erstreckt,
**dadurch gekennzeichnet,**
**dass** die Kanülenbasis (19) und der Kanülenmantel (18) zweiteilig ausgebildet sind und die Kanülenbasis (19) mit einem Stützarm (19a) nach vorne verlängert ist, der in einem nach vorne gerichteten Abstand (a) von der Kanülenbasis (19) eine quer gerichtete Abstützung zwischen diesen Teilen bildet.

14. Kanüle nach Anspruch 13,
**dadurch gekennzeichnet,**
**dass** die Kanülenbasis (19) und der Stützarm (19a) durch eine Steckverbindung mit einer Steckausnehmung (19c) und einem darin einfassenden Steckzapfen miteinander verbunden sind.

15. Kanüle nach Anspruch 13 oder 14,
**dadurch gekennzeichnet,**
**dass** der Stützarm (19a) mit einem Fußabschnitt (19b) und einer seitlich versetzten Wand (19e) winkelförmig geformt ist.

16. Kanüle nach Anspruch 15,
**dadurch gekennzeichnet,**
**dass** sich durch den Hohlraum der Winkelform jeweils ein oder mehrere Medienleitungen und/oder Lichtleiter (36a, 36b) erstrecken.
